## Europäisches Patentamt

## European Patent Office

## Office européen des brevets

(11) Veröffentlichungsnummer: **0 152 036**
**B1**

# EUROPÄISCHE PATENTSCHRIFT

(45) Veröffentlichungstag der Patentschrift:
25.10.89

(21) Anmeldenummer: **85101095.9**

(22) Anmeldetag: **02.02.85**

(51) Int. Cl.⁴: **C 12 P 19/24, C 12 N 11/14**

(54) Verfahren zur Herstellung von Isoglucose.

(30) Priorität: **13.02.84 DE 3405035**

(43) Veröffentlichungstag der Anmeldung:
**21.08.85 Patentblatt 85/34**

(45) Bekanntmachung des Hinweises auf die Patenterteilung:
**25.10.89 Patentblatt 89/43**

(84) Benannte Vertragsstaaten:
**AT BE CH DE FR GB IT LI LU NL SE**

(56) Entgegenhaltungen:
EP-A-0 000 028
EP-A-0 017 115
EP-A-0 017 176
EP-A-0 081 185
FR-A-2 346 366

STÄRKE, Band 36, Nr. 12, Dezember 1984, Seiten 412-416, Verlag Chemie GmbH, Weinheim, DE; G. WEIDENBACH et al.: "Glucose isomerase immobilized on SiO2-carrier with high productivity"

(73) Patentinhaber: **Kali- Chemie Aktiengesellschaft, Postfach 220 Hans- Böckler- Allee 20, D-3000 Hannover 1 (DE)**

(72) Erfinder: **Weidenbach, Günter, Dipl.- Chem. Dr.rer.nat., Lehrter Strasse 52, D-3000 Hannover 73 (DE)**
Erfinder: **Bonse, Dirk, Dr. Dipl.- Ing., Berliner Ring 3, D-3161 Arpke (DE)**
Erfinder: **Meyer, Boris, Dipl.- Chem. Dr.rer.nat., Schlesier Damm 18, D-3004 Isernhagen 2 (DE)**

(74) Vertreter: **Lauer, Dieter, Dr., c/o Kali- Chemie Aktiengesellschaft Postfach 220, D-3000 Hannover 1 (DE)**

**Beschreibung**

Die Erfindung betrifft ein Verfahren zur Herstellung einer Glucose und Fructose enthaltenden Lösung durch Umsetzen einer Glucose enthaltenden Lösung an einem auf der Basis $SiO_2$-Träger hergestellten Katalysator mit Glucoseisomeraseaktivität.

Ein derartiges Verfahren ist durch die DE-C-3 148 603 bekannt geworden. Gemäß die Produktivität bekannter, auf der Basis $SiO_2$-Träger hergestellter Trägerkatalysatoren mit Glucoseisomeraseaktivität erheblich gesteigert werden. Wenn die Glucose enthaltende Lösung vor der Umsetzung an dem Trägerkatalysator mit Formlingen aus $SiO_2$ oder Alumosilikat in Berührung gebracht wird.

Aufgabe der Erfindung ist es, andere Möglichkeiten zur Steigerung der Produktivität der bekannten $SiO_2$-Trägerkatalysatoren mit Glucoseisomeraseaktivität bereitzustellen.

Unter "auf der Basis $SiO_2$-Träger hergestellte Katalysatoren mit Glucoseisomeraseaktivität" bzw. unter "bekannten $SiO_2$-Trägerkatalysatoren mit Glucoseisomeraseaktivität" sind solche Katalysatoren zu verstehen, bei denen in einen oder auf einen Träger auf der Basis $SiO_2$, z. B. porösem Kieselgel, Glucoseisomerase eingearbeitet (z. B. durch Kopräzipitation) oder aufgebracht ist (z. B. durch adsorptive oder kovalente Bindung oder Vernetzung des Enzyms). Vorzugsweise sind darunter solche Katalysatoren zu verstehen, bei denen Glucoseisomerase an ein poröses Kieselgel adsorptiv oder kovalent gebunden oder durch Vernetzen aufgebracht ist, wobei gegebenenfalls auch Kombinationen derartiger Bindungsverfahren angewendet werden können. In diesem Sinne bevorzugte Katalysatoren sind beispielsweise aus der DE-C-2 726 188 bekannt.

Eine im Sinne der Aufgabenstellung andere Möglichkeit zur Steigerung der Produktivität besteht nun erfindungsgemäß darin, daß man der Glucose enthaltenden Lösung $SiO_2$ in Form eines wasserlöslichen Alkalimetallsilikats zusetzt.

Die zugesetzte Menge an $SiO_2$ ist nicht besonders kritisch. Um eine Produktivitätssteigerung im Ausmaß des bekannten Verfahrens gemäß der DE-C-3 148 603 zu erzielen, sollte die zugesetzte Menge 30 ppm nicht unterschreiten. Vorteilhafterweise setzt man zwischen 30 und 80 ppm $SiO_2$ der Glucoselösung zu.

Besonders einfach und preiswert erfolgt der Zusatz an $SiO_2$ dann, wenn man Wasserglas-Lösung, vorzugsweise Natronwasserglas-Lösung, verwendet.

Hinsichtlich der Steigerung der Produktivität erzielt man beim erfindungsgemäßen Arbeiten Werte, wie diese mit dem Verfahren gemäß der DE-C-3 148 603 auch erreicht werden. Überraschenderweise hat jedoch die Zugabe an $SiO_2$ anstatt der Vorkontaktierung mit Formlingen aus $SiO_2$ oder Alumosilikat einen vorteilhaften Einfluß auf die Stabilität des Katalysators bei Betriebsstillstand. Es hat sich nämlich gezeigt, daß beim Arbeiten nach bekannten Verfahren, sei es gemäß DE-C-3 148 603 oder anderen Verfahren, die Aktivität des Katalysators bei Betriebsstillstand, sei es wegen Stromausfall oder aus Gründen anderer Störungen, irreversibel geschädigt wird, wenn nicht dafür gesorgt wird, daß der Katalysator möglichst schnell auf Raumtemperatur abgekühlt wird. Zwar fallen beispielsweise bei einem Stromausfall nicht nur die Pumpen, sondern auch die Heizung für die Erwärmung des Substrats aus. Die im Katalysator und in der ihn umgebenden Substratlösung enthaltene Wärmekapazität verhindert jedoch das notwendige schnelle Abkühlen und reicht aus, den Katalysator zu schädigen. Um solche Schäden zu vermeiden, muß beispielsweise durch Notstromaggregate dafür Vorsorge getroffen werden, daß bei Betriebsstillstand kalte Substratlösung durch den Reaktor gepumpt werden kann, um so die notwendige schnelle Abkühlung des empfindlichen Katalysators zu erreichen. Beim erfindungsgemäßen Arbeiten können jedoch solche Vorsorgemaßnahmen völlig entfallen.

Die Ausführung des erfindungsgemäßen Verfahrens erfolgt bei insbesondere kontinuierlicher Arbeitsweise zweckmäßigerweise derart, daß man einer wäßrigen Glucoselösung mit einem Gehalt von etwa 40 bis 50 Gew.-% Trockensubstanz in der erfindungsgemäßen Weise $SiO_2$ zusetzt, die Lösung auf einen für die trägergebundene Glucoseisomerase geeigneten pH-Wert einstellt und auf eine für die trägergebundene Glucoseisomerase geeignete Isomerisierungstemperatur erwärmt. Die so vorbereitete Glucoselösung wird durch einen Reaktor gepumpt, der mit dem $SiO_2$-Trägerkatalysator mit Glucoseisomeraseaktivität gefüllt ist. Die vom Reaktor ablaufende Glucose-Fructose-Lösung wird kontinuierlich oder in regelmäßigen Abständen auf ihren Fructose-Gehalt untersucht, beispielsweise mittels polarimetrischer Analyse oder HPLC. Die Raumgeschwindigkeit (v/vh = Volumen Glucoselösung pro vom Katalysator in Anspruch genommenes Reaktorvolumen pro Stunde), mit der der Katalysator betrieben wird, wird dabei so eingestellt, daß die ablaufende Lösung, bezogen auf Trockensubstanz, einen innerhalb geringer Schwankungsbreiten von beispielsweise ± 1 %-Punkte konstanten Gehalt an Fructose von beispielsweise 42 Gew. % aufweist. Da in Abhängigkeit von Betriebszeit und insbesondere von der Betriebstemperatur der Katalysator ständig an Aktivität einbüßt, bedeutet das, daß die Raumgeschwindigkeit ständig zurückgenommen werden muß, um einen konstanten Isomerisierungsgrad zu erzielen. Der Isomerisierungsgrad gibt dabei, ausgedrückt in Prozent, die Anzahl der Moleküle Fructose an, die pro 100 in der Ausgangslösung enthaltenen Moleküle Glucose nach Durchströmen des Reaktors und Umsetzung an dem Katalysator in der ablaufenden Glucose-Fructose-Lösung enthalten sind. Je nach Reinheit bzw. Qualität der für die Herstellung der Glucoselösung eingesetzten Glucose muß, um den genannten Wert von 42 Gew.-% Fructose in der Trockensubstanz der ablaufenden Glucose-Fructose-Lösung zu erzielen, der Katalysator mit Raumgeschwindigkeiten betrieben werden, die einen Isomerisierungsgrad von etwa 44 bis 47 % erzielen lassen.

Falls bei der Herstellung des Trägerkatalysators mit Glucoseisomeraseaktivität eine Streptomyces albus Glucoseisomerase verwendet wurde, hat sich die Einstellung der Glucoselösung auf einen pH von etwa 7,0 bis 8,5 und eine Erwärmung der Glucoselösung auf eine Temperatur von etwa 55 bis 65°C als besonders günstig erwiesen.

Außerdem hat sich bei einer Streptomyces albus Glucoseisomerase die Zugabe von Co(II)- und Mg(II)-Ionen zur Glucoselösung in Mengen von etwa 0,1 bis 2 ppm Co(II) und etwa 10 bis 200 ppm Mg(II), zweckmäßigerweise in Form ihrer wasserlöslichen Salze wie beispielsweise Chloride oder Sulfate, als isomerisierungsfordernd erwiesen.

Schließlich ist es noch vorteilhaft, der Glucoselösung eine stabilisierende Menge eines Antioxidants, vorzugsweise $SO_2$ in einer Menge von etwa 100 bis 600 ppm in Form von Alkalimetallsulfit oder -bisulfit, hinzuzufügen.

Bevor die erhaltenen Glucose-Fructose-Lösung ihrer endgültigen Verwendung zugeführt wird, ist es noch empfehlenswert, die unerwünschten, weil beispielsweise geschmacksbeeinträchtigenden ionischen Komponenten mittels Kationen-und Anionenaustauschern aus der Glucose-Fructose-Lösung zu entfernen. Gegebenenfalls kann die Lösung noch zum Sirup eingeengt werden. Am Markt wird ein solcher Sirup unter Bezeichnungen wie Isosirup, Isomerose oder Isoglucose gehandelt.

Die nachfolgenden Beispiele dienen der Erläuterung der Erfindung.

## Beispiel 1

### Allgemeines Beispiel

Für alle Beispiele wurde als "auf der Basis $SiO_2$-Träger hergestellter Katalysator mit Glucoseisomeraseaktivität" ein gemäß DE-C-2 726 188 hergestellter Katalysator verwendet. Jeweils 5 g dieses Katalysators wurden in einen Reaktor gefüllt. Durch den Reaktor strömte eine auf 60°C erwärmte Glucoselösung. Die Einstellung der Raumgeschwindigkeit (bezogen auf das den Katalysator enthaltende Reaktorvolumen) erfolgte so, daß der Isomerisierungsgrad über die gesamte Betriebszeit konstant 46,5 % betrug. Der Isomerisierungsgrad der ausfließenden Substratlösung wurde polarimetrisch gemessen. Im einzelnen sind Katalysator und Verfahren durch folgende wesentliche Parameter gekennzeichnet:

Katalysator:

| | |
|---|---|
| Träger | $SiO_2$ |
| Aktivitätsaufnahme | 9000 U/g |
| Korngröße | 0,1 - 0,2 mm |
| Schüttgewicht (trocken) | 0,45 kg/l |

Verfahren:

| | |
|---|---|
| Substrat | 45 Gew.-% Glucose in wäßriger Lösung |
| Kofaktoren | 120 ppm Mg (II) |
| | 1 ppm Co (II) |
| | 200 ppm $SO_2$ (in Form von $Na_2SO_3$ |
| pH-Wert | 7,5 |
| Substratdichte | 1,2 kg/l |
| Substrateingangstemperatur | 60°C |
| Isomerisierungsgrad | 46,5 % |
| Anfangsraumgeschwindigkeit | 13,0 h$^{-1}$ |

Aktivitätsbestimmung der Glucoseisomeraselösung:

Die Aktivität der zur Präparation des Katalysators eingesetzten Glucoseisomeraselösung wurde nach der Takasaki-Methode (vgl. Y. Takasaki: Agr. Biol. Chem., Vol. 30, 1966, Nr. 12, 1247-1253, und Z. Dische und E. Borenfreund: J.-Biol. Chem., 192, 583, 1951) bestimmt. Eine Aktivitätseinheit (U) ist definiert als die Enzymmenge, die unter Inkubationsbedingungen 1 mg Fructose bildet.

Inkubationsbedingungen:

| | |
|---|---|
| Temperatur | 65°C |
| Reaktionszeit | 1 h |
| Substrat | 0,1 m Glucose x $H_2O$ (Merck 8342) in 0,05 m Phosphatpuffer, pH 8,0 mit 0,0004 m $MgSO_4$ |

Maßgebliche Kriterien für die Qualität des Katalysators bzw. des Verfahrens, unter dem der Katalysator eingesetzt wird, sind neben der Halbwertszeit für die Aktivitätsabnahme, der Betriebszeit bis zur Restaktivität von 20 % der Ausgangsaktivität (20 % Restaktivität bezogen auf Ausgangsaktivität = 100 % haben sich als untere Grenze der noch wirtschaftlich verwertbaren Katalysatoraktivität erwiesen) vor allem die Produktivität. Die Produktivität ist dabei definiert als diejenige Substratmenge, berechnet als Trockensubstanz in kg, die bei einem vorgegebenen Isomerisierungsgrad von 1 kg Katalysator bis zu einer Restaktivität von 20 % der Ausgangsaktivität verarbeitet werden kann.

**Beispiel 1.1**

**Erfindungsgemäßes Beispiel**

Das allgemeine Beispiel wurde mit der Maßgabe ausgeführt, daß die Glucoselösung erfindungsgemäß 50 ppm $SiO_2$ in Form von Natronwasserglaslösung enthielt. Die Ergebnisse waren folgende:

| | |
|---|---|
| Halbwertszeit | 1300 h |
| Betriebszeit bis Restaktivität 20 % | 3800 h |
| Produktivität nach 3800 h | 26000 kg TS mit 46,5 Gew.-% Frucktose/1 kg Katalysator |

**Beispiel 1.2**

**Vergleichsbeispiel gemäß DE-C-3 148 603**

Das allgemeine Beispiel wurde mit der Maßgabe ausgeführt, daß die Glucoselösung nicht den erfindungsgemäßen Zusatz an $SiO_2$ enthielt, daß die Lösung jedoch gemäß DE-C-3 148 603 vor der Aufgabe auf den Reaktor durch eine dem Reaktor vorgeschaltete Vorsäule gepumpt wurde. Die Vorsäule war mit 5 g eines kommerziell erhältlichen, kugelförmigen, wasserfesten, porösen Alumosilikat (Zusammensetzung etwa 97 Gew.-% $SiO_2$ und 3 Gew.-% $Al_2O_3$; Korngröße 1-2 mm, Schüttgegewicht, trocken, 0,7 kg/l; Typ KCT-WS der Kali-Chemie AG) gefüllt.

Die erhaltenen Ergebnisse (Halbwertszeit, Betriebszeit, Produktivität) waren mit denen von Beispiel 1.1 identisch.

**Beispiel 1.3**

**Vergleichsbeispiel**

Dieses Beispiel entspricht dem allgemeinen Beispiel 1, d.h. die Glucoselösung enthielt weder den erfindungsgemäßen Zusatz an $SiO_2$ noch wurde die Glucoselösung über eine mit Formlingen aus $SiO_2$ oder Alumosilikat gefüllte Vorsäule geleitet. Die Ergebnisse sind folgende:

| | |
|---|---|
| Halbwertszeit | 670 h |
| Betriebszeit bis Restaktivität 20 % | 1700 h |
| Produktivität nach 1700 h | 12500 kg TS mit 46,5 Gew.-% Fructose/1 kg Katalysator |

**Beispiel 2**

Zwecks Simulation eines Betriebsstillstandes wurden die Beispiele 1.1, 1.2 und 1.3 wiederholt mit der Maßgabe, daß nach 300 Betriebsstunden der Durchfluß der Glucoselösung ohne Änderung der Reaktortemperatur für 3 Stunden unterbrochen wurde. Dies zeigte folgende Auswirkungen:

**Beispiel 2.1**

Gegenüber dem Beispiel 2.1 ergaben sich keine Veränderungen.

**Beispiel 2.2**

Nach Wiederaufnahme des Durchflusses war die Aktivität des Katalysators von 81 % auf 64 % der Anfangsaktivität abgefallen, was einem relativen Aktivitätsverlust von 21 % entsprach. Im übrigen wurden folgende Endergebnisse erhalten:

| | |
|---|---|
| Halbwertszeit | 800 h |
| Betriebszeit bis Restaktivität 20 % | 2900 h |
| Produktivität nach 2900 h | 19200 kg TS mit 46,5 Gew.-% Fructose/1 kg Katalysator |

**Beispiel 2.3**

Bei diesem Beispiel war nach Wiederaufnahme des Durchflusses die Aktivität des Katalysators von 75 % auf 59 % der Anfangsaktivität abgefallen, was einem relativen Aktivitätsverlust von ebenfalls 21 % entspricht. Die Endergebnisse zeigten folgende Werte:

| | |
|---|---|
| Halbwertszeit | 480 h |
| Betriebszeit bis Restaktivität 20 % | 1300 h |
| Produktivität nach 1300 h | 9600 kg TS mit 46,5 Gew.-% Fructose/1 kg Katalysator |

Die Beispiele zeigen, daß mit der Erfindung eine gleichgute Produktivitätssteigerung wie beim Arbeiten gemäß der DE-C-3 148 603 erzielt werden kann, daß jedoch beim erfindungsgemäßen Arbeiten Betriebsunterbrechungen selbst bei Konstanthaltung der Reaktortemperatur keine Schädigung der Katalysatoraktivität zur Folge haben.

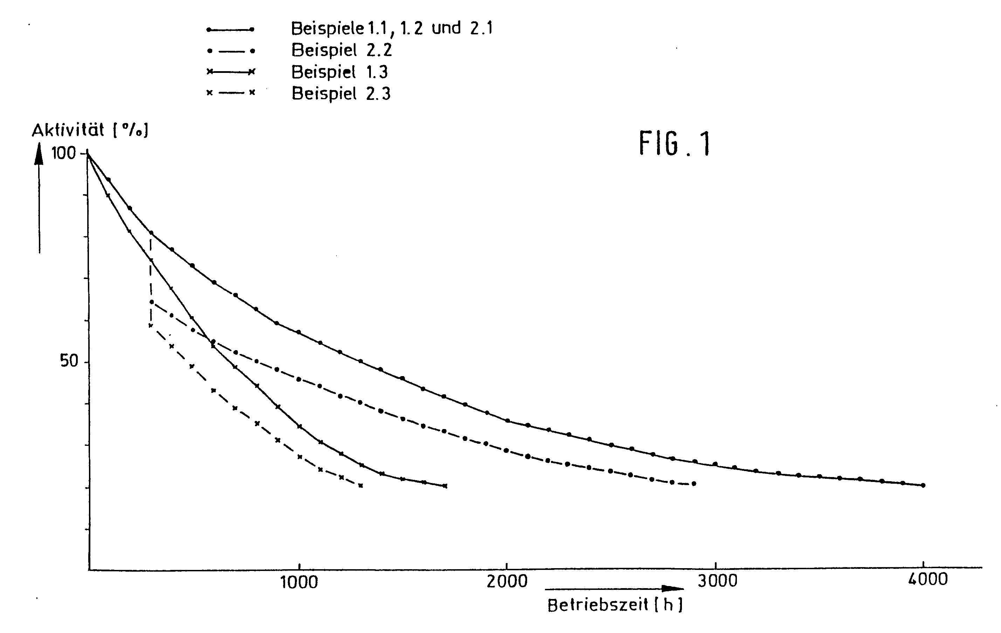

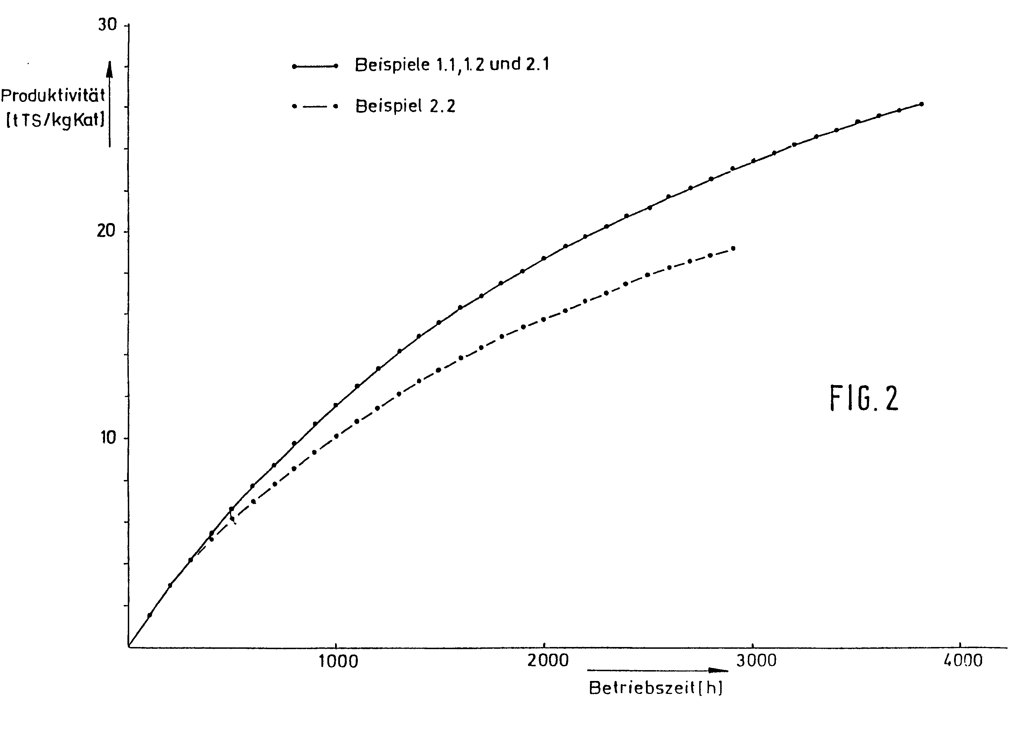

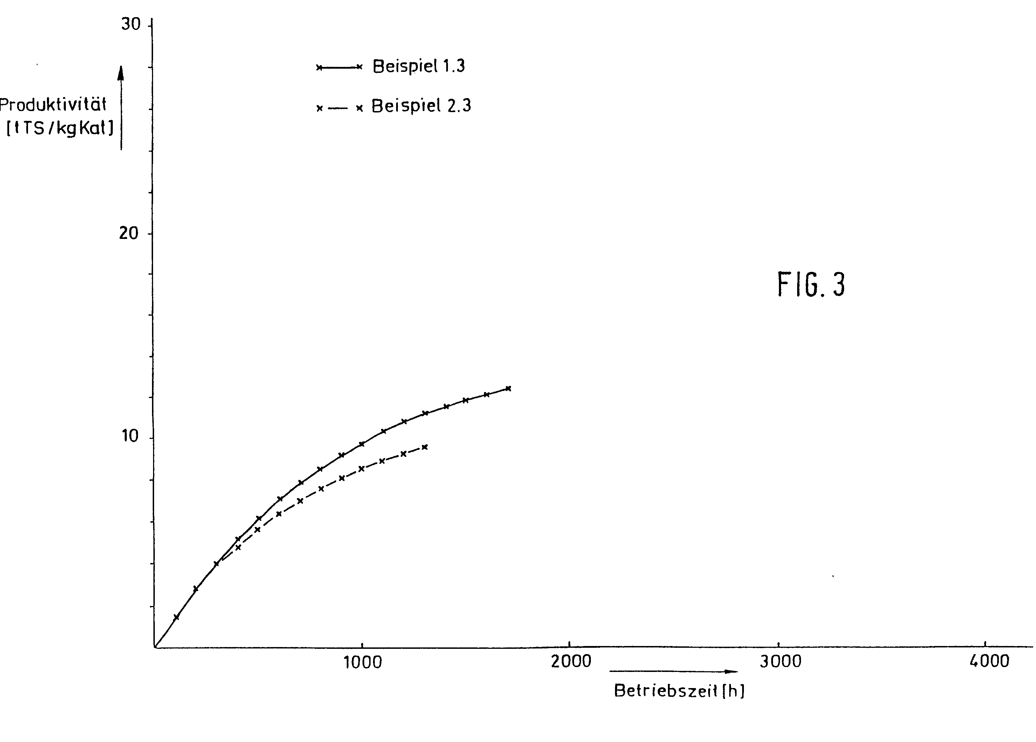

Zur Veranschaulichung der Beispiele wurden in den nachfolgenden Diagrammen die Aktivitätsabnahme (Abb. 1) sowie die Entwicklung der Produktivität (Abb. 2 und 3), jeweils in Abhängigkeit von der Betriebszeit dargestellt. In allen Diagrammen ist auf der Abszisse die Betriebszeit in Stunden aufgetragen. Auf der Koordinate der Abb 1. ist die Aktivität in % und auf der Koordinate der Abb. 2 und 3 ist die Produktivität in t (Tonnen) Trockensubstanz (TS) pro kg Katalysator aufgetragen. Die Meßwerte für die einzelnen Kurven sind wie folgt dargestellt.

| | |
|---|---|
| Beispiele 1.1. 1.2 und 2.1 | durch ".___." |
| Beispiel 1.3 | durch "x___x" |
| Beispiel 2.2 | durch ".--." |
| Beispiel 2.3 | durch "x--x" |

**Patentansprüche**

1. Verfahren zur Herstellung einer Glucose und Fructose enthaltenden Lösung durch Umsetzen einer Glucose enthaltenden Lösung an einem auf der Basis $SiO_2$-Träger hergestellten Katalysator mit Glucoseisomeraseaktivität, dadurch gekennzeichnet, daß man der Glucoselösung $SiO_2$ in Form eines wasserlöslichen Alkalimetallsilikats zusetzt.

2. Verfahren nach Anspruch 1, dadurch gekennzeichnet, daß man der Glucoselösung $SiO_2$ in einer Menge von 30 bis 80 ppm zusetzt.

3. Verfahren nach Anspruch 1 oder 2, dadurch gekennzeichnet, daß man der Glucoselösung $SiO_2$ in Form von Wasserglas-Lösung zusetzt.

**Claims**

1. A method for preparing a solution containing glucose and fructose by reacting a glucose-containing solution on a catalyst with glucose isomerase activity produced on the basis of $SiO_2$ carriers, characterised in that $SiO_2$ is added to the glucose solution in the form of a water-soluble alkali metal silicate.

2. A method according to claim 1, characterised in that $SiO_2$ is added to the glucose solution in a quantity of 30 to 80 ppm.

3. A method according to claim 1 or 2, characterised in that $SiO_2$ is added to the glucose solution in the form of waterglass solution.

**Revendications**

1. Procédé pour la préparation d'une solution contenant du fructose et du glucose par réaction d'une solution contenant du glucose sur un catalyseur sur un support à base de $SiO_2$ doué d'une activité glucose isomérase, caractérisé en ce qu'on ajoute à la solution de glucose du $SiO_2$ sous forme d'un silicate de métal alcalin hydrosoluble.

2. Procédé selon la revendication 1, caractérisé en ce qu'on ajoute à la solution de glucose du $SiO_2$ à raison de 30 à 80 ppm.

3. Procédé selon la revendication 1 ou 2, caractérisé en ce qu'on ajoute à la solution de glucose du $SiO_2$ sous la forme d'une solution de verre soluble.

Beispiele 1.1, 1.2 und 2.1
Beispiel 2.2
Beispiel 1.3
Beispiel 2.3

FIG. 1

Aktivität [%]

100

50

1000    2000    3000    4000

Betriebszeit [h]

EP 0 152 036 B1

FIG. 2

EP 0 152 036 B1

FIG. 3